# EUROPEAN PATENT APPLICATION

(11) **EP 4 364 763 A1**
(43) Date of publication of application: **08.05.2024**
(21) Application number: 22832532.0
(22) Date of filing: 28.03.2022
(51) Int. Cl.: A61M 1/16

(54) **BLOOD PURIFICATION DEVICE**

(30) Priority: 30.06.2021 JP 2021109353
(71) Applicant: Nikkiso Co., Ltd., Tokyo 150-6022 (JP)
(72) Inventor: OBATA, Yuki, Makinohara-shi, Shizuoka 421-0496 (JP)
(74) Representative: von Hirschhausen, Helge
(86) International application number: PCT/JP2022/015072
(87) International publication number: WO 2023/276371

(57) **Abstract**

An object is to provide a blood purification apparatus which can easily perform heating and quickly complete a disinfection process. A blood purification apparatus (100) includes a blood circuit (110), a dialysis circuit (120), and a dialyzer (130). A control circuit (10) causes disinfection liquid inside the dialysis circuit to flow by means of a dual pump (135) while heating the disinfection liquid by supplying stored power from a battery for returning blood in addition to commercial power to a heater (125) to generate heat.

## Description

### Technical Field

The present invention relates to a blood purification apparatus that draws out blood into an external blood circuit and returns the blood while purifying the blood.

### Background Art

In so-called "dialysis treatment" which is a treatment for purifying blood, a apparatus that purifies blood by dialysis while circulating the blood through an extracorporeal blood circuit is often used. Such a apparatus guides the blood of each person into a blood circuit and returns the blood to the person while performing dialysis using a dialysate. For this reason, in a blood purification apparatus, even if a blood circuit that circulates blood is replaced each time, a dialysis circuit to which various equipment is attached and which circulates the dialysate must be cleaned to a high level and repeatedly used, and it is necessary to perform disinfection or the like before starting the treatment (for example, see Patent Literatures 1 and 2).

### Citation List

### Patent Literature

Patent Literature 1: Japanese Patent Laid-Open No. 2014-97197
Patent Literature 2: Japanese Patent Laid-Open No. 2017-74086

### Summary of Invention

### Technical Problem

However, in such a blood purification apparatus, when heating a disinfection liquid to around the level of boiling water, heating with a small heater takes a long time, and furthermore, to install a large heater it is necessary to change the design of the current-carrying capacity of the apparatus body, and in some cases it may be necessary to perform construction work to augment the power supply of the facility where treatment is performed.

Therefore, an object of the present invention is to provide a blood purification apparatus that can easily perform heating and quickly complete a disinfection process.

### Solution to Problem

One aspect of the invention of a blood purification apparatus that solves the above problem is a blood purification apparatus including a blood purifier, the blood purification apparatus including: a disinfector that causes a disinfection liquid to flow along a flow route of the blood purifier to disinfect the flow route; a heating unit that heats the disinfection liquid; and a controller that controls a blood purification treatment of the blood purifier and also controls a disinfection process of the disinfector and a heating process of the heating unit; wherein the heating unit heats the disinfection liquid by being supplied with electric power from an auxiliary power source and generating heat in addition to being supplied with electric power from a commercial power source and generating heat.

### Advantageous Effect of Invention

As described above, according to one aspect of the present invention, a blood purification apparatus that can easily perform heating and quickly complete a disinfection process can be provided.

### Brief Description of Drawings

[Figure 1] Figure 1 is a view illustrating a blood purification apparatus according to a first embodiment of the present invention, and is a circuit diagram illustrating a schematic overall configuration of the blood purification apparatus.
[Figure 2] Figure 2 is a circuit diagram illustrating a circuit when subjecting a dialysis circuit of the blood purification apparatus to a disinfection process.
[Figure 3] Figure 3 is a block diagram illustrating a configuration that executes control processing of the blood purification apparatus.
[Figure 4] Figure 4 is a graph for describing temperature changes when subjecting the dialysis circuit to a disinfection process.
[Figure 5] Figure 5 is a process chart for describing the disinfection process performed on the dialysis circuit.
[Figure 6] Figure 6 is a timing chart for describing the disinfection process, for each disinfection step, performed on the dialysis circuit.
[Figure 7] Figure 7 is a block diagram of essential parts that illustrates another form of a heater of the blood purification apparatus.
[Figure 8] Figure 8 is a multi-view drawing illustrating a blood purification apparatus according to a second embodiment of the present invention, in which (A) is a partially cutaway plan view illustrating the configuration of a heater thereof, and (B) is a circuit diagram for each resistance component thereof.
[Figure 9] Figure 9 is a block diagram illustrating a configuration that executes control processing of the blood purification apparatus.
[Figure 10] Figure 10 is a view illustrating a blood purification apparatus according to a third embodiment of the present invention, and is a circuit diagram illustrating a circuit when subjecting a dialysis circuit thereof to a disinfection process.

### Description of Embodiments

Hereunder, embodiments of the present invention are described in detail with reference to the accompanying drawing.

### <First Embodiment>

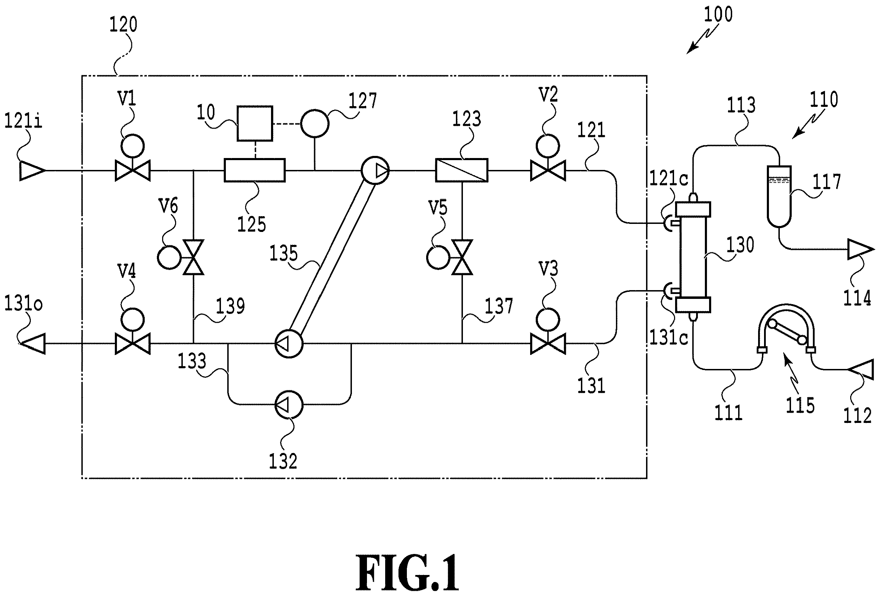

Figure 1 to Figure 6 are views illustrating a blood purification apparatus according a first embodiment of the present invention.

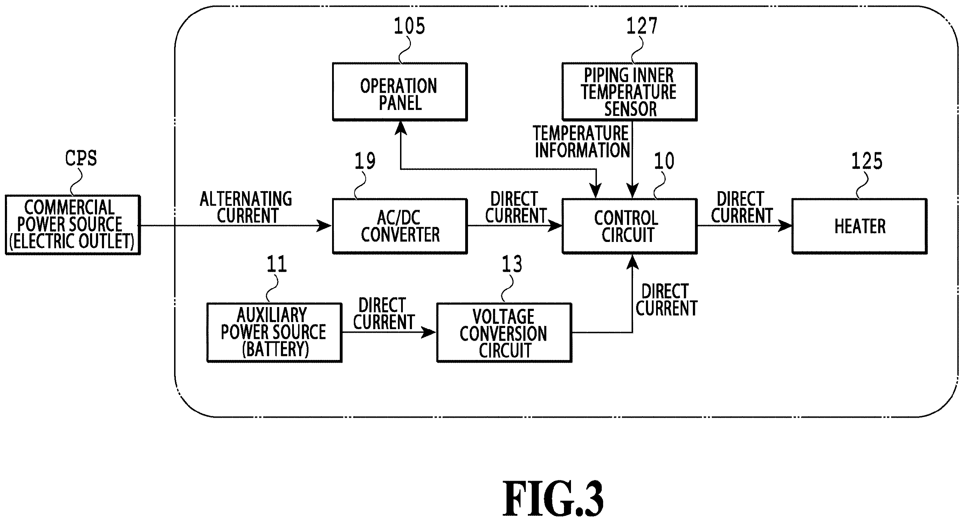

In Figure 1, a blood purification apparatus 100 includes a blood circuit 110, a dialysis circuit 120, and a dialyzer 130, and is constructed so as to function as means for purifying the blood of a patient. The blood purification apparatus 100 is constructed as a medical apparatus which, by a control circuit (controller) 10 executing a control program stored in advance in a memory, performs a dialysis treatment (blood purification treatment) by driving respective parts of the apparatus including the blood circuit 110 and the dialysis circuit 120 based on an input operation from an operation panel 105 (see Figure 3) and various kinds of sensor information and the like to cause the blood of the patient to pass through the dialyzer 130 to dialyze and purify the blood.

The blood circuit 110 is constructed so that the dialyzer 130 is connected so as to be interposed between a blood removal line 111 and a blood return line 113 which are flexible tubes, and so that the blood of the patient passes through the dialyzer 130. In the blood circuit 110, a connector 112 for connecting an arterial-side puncture needle for puncturing an artery of a patient is attached to the tip of the blood removal line 111 that is connected to one end side of the dialyzer 130, and a connector 114 for connecting a venous-side puncture needle for puncturing a vein of a patient is attached to the tip of the blood return line 113 that is connected to the other end side of the dialyzer 130. Further, a blood pump 115 that is a tubing pump is arranged on the blood removal line 111 side, and an air trap chamber 117 is arranged on the blood return line 113 side.

In the blood circuit 110, when the blood pump 115 on the blood removal line 111 side is driven by the control circuit 10, blood that is drawn out and removed from the arterial side of the patient is dialyzed and purified by passing through the dialyzer 130, and thereafter blood that has undergone a bubble removal treatment for removing dissolved air by the air trap chamber 117 on the blood return line 113 side is returned to the venous side of the patient.

The dialysis circuit 120 is constructed so as to be connected by connecting tools 121c and 131c to the dialyzer 130 in a manner so that the dialyzer 130 is interposed between an introduction line 121 and a drainage line 131 which are flexible tubes, and causes a dialysate of a predetermined concentration to pass therethrough to subject the blood of the patient to a dialysis and purification treatment. In the dialysis circuit 120, solenoid valves V1 and V2 are arranged along with a filtration filter 123, a heater (heating unit) 125, and a thermistor (temperature sensor) 127 on the introduction line 121 side. Further, on the drainage line 131 side, solenoid valves V3 and V4 are arranged as well as a detour line 133 equipped with a water removal pump 132. In the dialysis circuit 120, a dual pump 135 is arranged so as to straddle between the introduction line 121 and the drainage line 131, and bypass lines 137 and 139 in which solenoid valves V5 and V6 are provided, respectively, are also arranged therein.

The dialysis circuit 120 is configured so that while dialysate adjusted to a predetermined concentration is sucked in from an inlet 121i of the introduction line 121 and sent to the dialyzer 130 through the filtration filter 123 by the control circuit 10 driving the dual pump 135, dialysate containing waste products and the like resulting from dialysis by the dialyzer 130 is drained from an outlet 131o. The dialysis circuit 120 is constructed so as to subject blood that flows through the dialyzer 130 to a water removal treatment by the control circuit 10 driving the water removal pump 132 of the detour line 133.

At such time, in the dialysis circuit 120, the control circuit 10 energizes the heater 125 based on temperature information pertaining to the dialysate in the introduction line 121 measured by the thermistor 127 to activate the heater 125 to generate heat, to thereby heat the dialysate in the introduction line 121 and maintain a dialysis treatment temperature suitable for blood purification treatment, which is about the body temperature of the patient. Here, the solenoid valves V1 to V6 are operated to open and close by the control circuit 10 so as to block or open flow routes in each line at any desired timing.

The dialyzer 130 is connected to the introduction line 121 and the drainage line 131 of the blood circuit 110, and flow routes which the dialysate flows into and is caused to flow through are formed in hollow fibers (not shown) inside the dialyzer 130. The dialyzer 130 performs dialysis that separates waste products, excessive water and the like contained in the blood of the patient flowing within the hollow fibers by utilizing micropores in a blood purification membrane thereof to purify the blood of the patient. Because of having such a structure, the blood circuit 110 is used in a disposable manner by removing the blood removal line 111 and the blood return line 113 contaminated with the blood of the patient from the blood pump 115 that is a tubing pump and the like, and detaching the blood removal line 111 and the blood return line 113 together with the dialyzer 130.

On the other hand, the dialysis circuit 120 is constructed to include the dual pump 135 and the like and mechanisms such as the solenoid valves V1 to V6 along with the heater 125 and the filter 123 and the like, and causes the dialysate to flow therethrough, and therefore the dialysis circuit 120 is generally cleaned by disinfection or the like and repeatedly used.

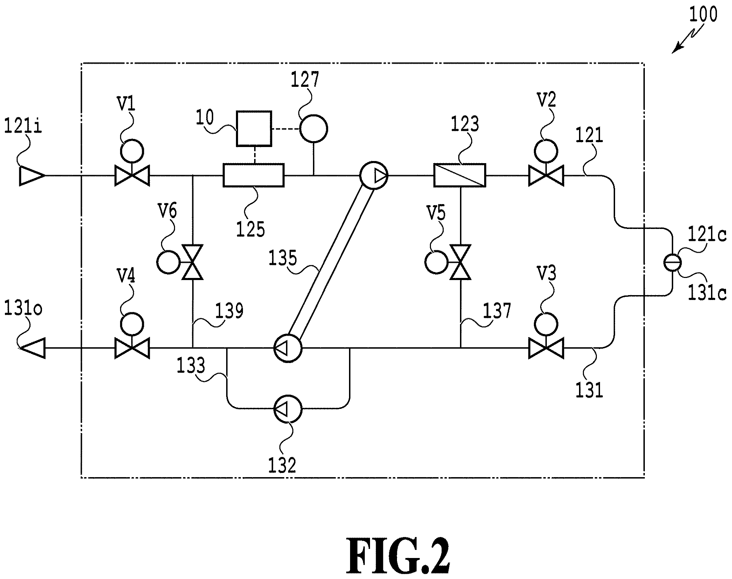

Therefore, as illustrated in Figure 2, a closed circuit is formed in the dialysis circuit 120 by connecting the connecting tools 121c and 131c of the introduction line 121 and the drainage line 131 to each other, and the dialysis circuit 120 is configured to execute a disinfection process that circulates a disinfection liquid by the control circuit 10 closing the solenoid valves V1 and V4 and opening the solenoid valve V6 while driving the dual pump 135 and the water removal pump 132 to form a flow route. At such time, the dialysis circuit 120 is configured to execute the disinfection process by appropriately switching the circulation route so as to pass through the bypass line 137 or the connecting tools 121c and 131c, by the control circuit 10 opening and closing the solenoid valves V2, V3, and V5 at arbitrary timings.

Specifically, as illustrated in Figure 3, the blood purification apparatus 100 is configured so as to perform dialysis by converting alternating current power from a commercial power source CPS to which the blood purification apparatus 100 is connected through an electric outlet to direct current through an AC/DC converter 19, and supplying the electric power to each part of the apparatus including the control circuit 10. For example, the control circuit 10 energizes and actuates the heater 125 based on temperature information pertaining to the disinfection liquid in the introduction line 121 of the dialysis circuit 120 measured by the thermistor 127, to thereby raise the temperature of the disinfection liquid in the introduction line 121 and maintain the disinfection liquid at a temperature that is suitable for a disinfection process that disinfects by causing the disinfection liquid to flow through flow routes of the introduction line 121 and the drainage line 131 as boiling water.

Further, a battery (auxiliary power source) 11 as an auxiliary power source is mounted in the blood purification apparatus 100. For example, during a power outage, the control circuit 10 receives stored power from the battery 11 and drives the blood pump 115 to execute a blood return process that returns the blood to the patient. Note that, the power stored in the battery 11 is adjusted to a desired voltage by a voltage conversion circuit 13 so that power is appropriately supplied. Here, although a case of supplying power from the battery 11 is described as one example in the present embodiment, the present invention is not limited thereto, and it need scarcely be said that a configuration may be adopted that supplies power from a power generator or other peripheral equipment.

Further, when performing a treatment to disinfect the dialysis circuit 120, the control circuit 10 is configured to rapidly raise the temperature of the disinfection liquid in the introduction line 121 by utilizing power stored in the battery 11 in addition to electric power from the commercial power source (hereinafter, also referred to as "commercial power"), and is configured to parallelly execute a process to store power in the battery 11 while maintaining the disinfection liquid at a desired temperature for a desired period with commercial power after raising the temperature of the disinfection liquid. That is, the heater 125 functions as a shared heater unit that is supplied with commercial power and stored power from the battery 11 and generates heat.

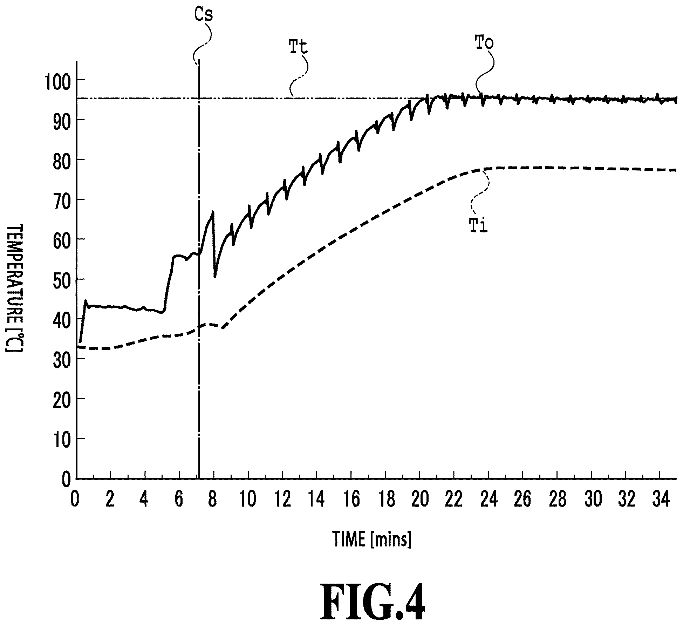

As illustrated in Figure 4, because the start of a rise in the temperature of the disinfection liquid in the introduction line 121 is delayed relative to the timing at which the heater 125 is energized, the control circuit 10 is configured to circulate the disinfection liquid including through the drainage line 131 while causing the temperature of the disinfection liquid to rapidly rise in a manner that takes the timing at which the temperature of the disinfection liquid starts to rise as a disinfection process start timing Cs of the dialysis circuit 120. At such time, the control circuit 10 is configured to adjust the power supply to the heater 125 so that a temperature To immediately after the heater 125 in the introduction line 121 does not exceed a target temperature Tt so as not to cause the disinfection liquid to boil. Further, since a rise in a disinfection liquid temperature Ti is delayed at a place located away from the heater 125 in the dialysis circuit 120, the control circuit 10 is configured to refer to preset heating conditions with which a sufficient disinfection effect can be obtained from a time point at which the temperature To near the heater 125 reaches the target temperature Tt, and maintain a desired temperature or higher for a desired time period. Specifically, the control circuit 10 adjusts so that the disinfection liquid which is being heated does not boil in order that the pressure in the dialysis circuit 120 does not rapidly increase and damage the dialysis circuit 120, for example, in a similar manner to pulse width modulation (PWM), by adjusting the duration of a supplying time period and a stopping time period according to the temperature difference to reduce the amount of energization as the temperature To near the heater 125 approaches the target temperature Tt. That is, together with the heater 125 that heats the disinfection liquid, the control circuit 10 and the dual pump 135 for circulating the disinfection liquid in the dialysis circuit 120 constitute a disinfector.

Here, the control circuit 10 supplies (energizes) the heater 125 with commercial power and stored power from the battery 11 to heat and raise the temperature of the disinfection liquid in the introduction line 121 so as to satisfy the set heating conditions, and in this case a configuration may be adopted so as to perform heating to raise the temperature of the disinfection liquid for a set time period which allows some margin to spare depending on the performance of the heater 125 so as to satisfy the desired temperature or higher for the desired period or longer. Further, at such time the control circuit 10 may be configured to, for example, monitor the temperature detected by the thermistor 127 and transition to the next treatment process at a timing at which the desired temperature or higher is satisfied for the desired period, to thereby quickly and reliably complete the disinfection process on the dialysis circuit 120 in a manner that avoids insufficient heating as well as wasted expense occurring due to an excessive heating time or excessive electric power supply because of the influence of the room temperature, water temperature, voltage fluctuations and the like.

In addition, the control circuit 10 is configured to detect one or both of the voltage and the discharge current to thereby ascertain (detect) the remaining amount of stored electricity in the battery 11, and is configured to prevent deterioration due to overdischarge of the battery 11 by stopping the discharge (supply) of the power stored in the battery 11 and cause the heater 125 to perform heating operations using only the commercial power before the amount of electricity stored in the battery 11 is exhausted. In this case, the control circuit 10 is configured to display and output various information such as information indicating that the battery 11 is exhausted or that the heating time is extended on the operation panel 105 (a buzzer or an indicator lamp or the like may also be used) to thereby notify a user to prompt the user who ascertained that there is a reduction in the amount of stored electricity in the battery 11 to check if there is a deterioration in the storage capacity or the like and take countermeasures. By this means, the effective use of the battery 11 for a long period of time can be ensured with high reliability, and early replacement of the battery 11 can be avoided to thereby prevent an increase in cost.

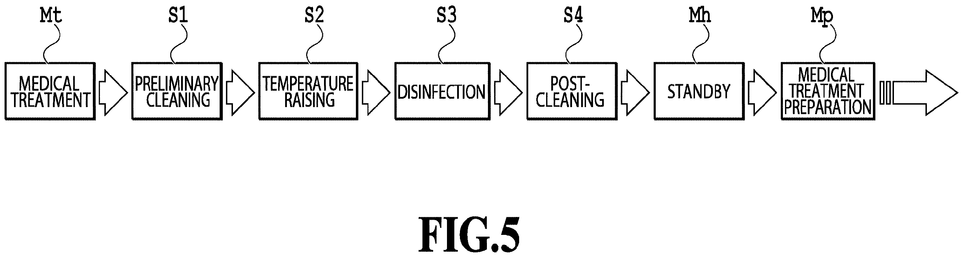

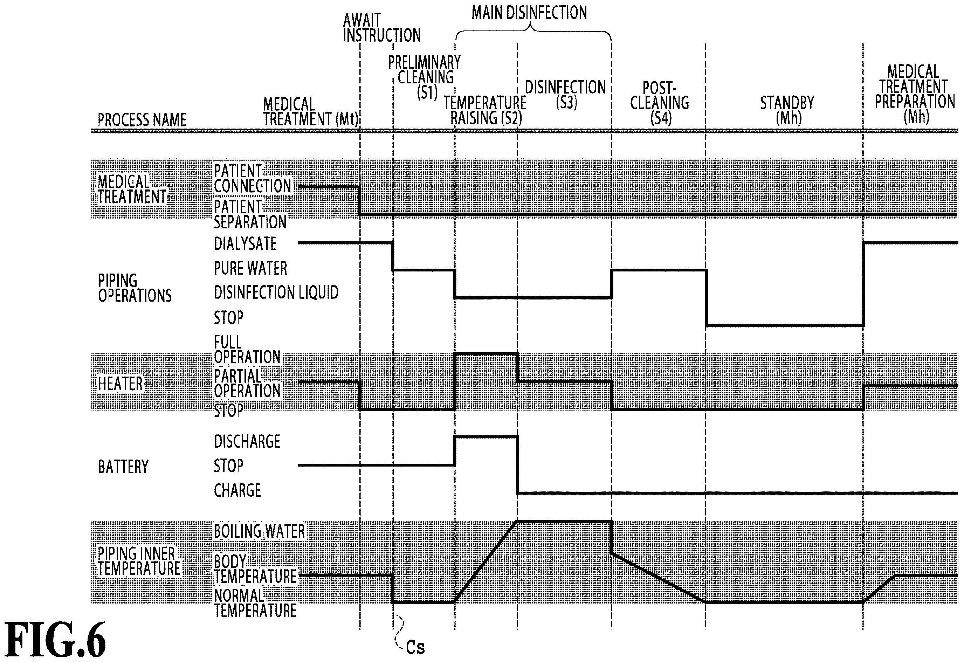

More specifically, as illustrated in Figure 5 and Figure 6, the control circuit 10 is configured to execute a control program in the memory in accordance with an instruction input from the operation panel 105 after the end of the medical treatment to subject the dialysis circuit 120 to an automatic disinfection process (disinfection method).

First, when a medical treatment (Mt) in which the blood circuit 110 is connected to a patient to perform dialytic purification of the blood is completed, and the blood circuit 110 is disconnected from the patient and a closed circuit of the dialysis circuit 120 is formed and an input operation that instructs the end (Ce) of the medical treatment is performed from the operation panel 105, the control circuit 10 performs processing to stop each part of the apparatus including a partial operation for heating using commercial power by the heater 125.

At such time, it is regarded by the control circuit 10 that an instruction to perform an automatic disinfection process with respect to the dialysis circuit 120 has been input, and the control circuit 10 performs a preliminary cleaning step (S1) in which the used dialysate in the dialysis circuit 120 is replaced with pure water, and the pure water is circulated for a predetermined time period (set time period). In the preliminary cleaning, power is not supplied from the battery 11 or the commercial power source, and the heater 125 remains in a stopped state, and the temperature along the route in the dialysis circuit 120 changes from the temperature around body temperature of the dialysate during the medical treatment to normal temperature when the used dialysate is replaced with pure water.

Thereafter, the control circuit 10 performs a main disinfection in which the pure water that was used for preliminary cleaning in the dialysis circuit 120 is replaced with a disinfection liquid such as citric acid, and raises the temperature thereof and circulates the disinfection liquid to disinfect the dialysis circuit 120. In this main disinfection, a temperature raising step (S2) is executed in which full operation for supplying commercial power and also supplying power stored in the battery 11 to the heater 125 is performed to rapidly raise the temperature of the disinfection liquid in the dialysis circuit 120 to the target temperature, and thereafter a circulatory disinfection step (S3) is executed in which partial operation for supplying only commercial power to the heater 125 for heating is performed to maintain the disinfection liquid inside the dialysis circuit 120 at a desired temperature for a predetermined time period. From this circulatory disinfection step (S3) onwards, a charging process for charging the battery 11 in parallel with other processes is started. Here, although a case where citric acid is used as the disinfection liquid is described as one example in the present embodiment, the present invention is not limited thereto, and it need scarcely be said that, for example, the disinfection may be disinfection with boiling water that is pure water or may be hypo disinfection.

Next, the control circuit 10 performs a post-cleaning step (S4) in which the used disinfection liquid in the dialysis circuit 120 is replaced with pure water and the pure water is circulated for a predetermined time period. Thereafter the control circuit 10 drains the pure water used for the post-cleaning from the dialysis circuit 120 and stands by (Mh). In the post-cleaning, the temperature along the route in the dialysis circuit 120 is gradually lowered to normal temperature by replacing the disinfection liquid which is at a high temperature with pure water which is at normal temperature.

Thereafter, in accordance with the medical treatment schedule for another patient, the control circuit 10 supplies dialysate to the dialysis circuit 120, and also supplies only commercial power to the heater 125 to perform a partial operation to heat the heater 125 to thereby perform medical treatment preparation (Mp).

### <Advantageous Effect of First Embodiment>

As described above, in the blood purification apparatus 100 of the present embodiment, when subjecting the dialysis circuit 120 to a disinfection process, the disinfection liquid can be heated by also supplying electric power from the battery 11 to the heater 125 in addition to electric power from a commercial power source, and hence the disinfection liquid can be easily heated and the disinfection process can be quickly completed.

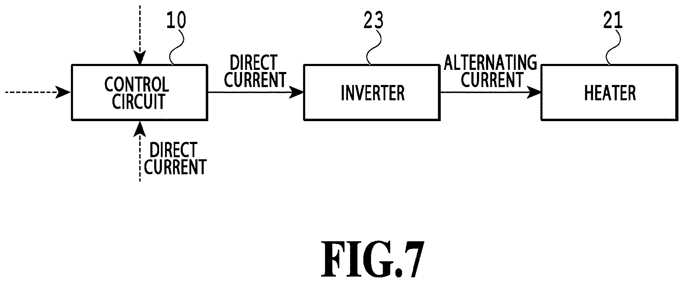

Here, although a case where the DC heater 125 is used as a shared heater is described as an example in the present embodiment, the present invention is not limited thereto, and it need scarcely be said that an AC heater may be used. In such case, as illustrated in Figure 7, it suffices to adopt a circuit configuration in which direct current power that the control circuit 10 outputs is converted by an inverter 23 and supplied as alternating current power to an AC heater 21.

### <Second Embodiment>

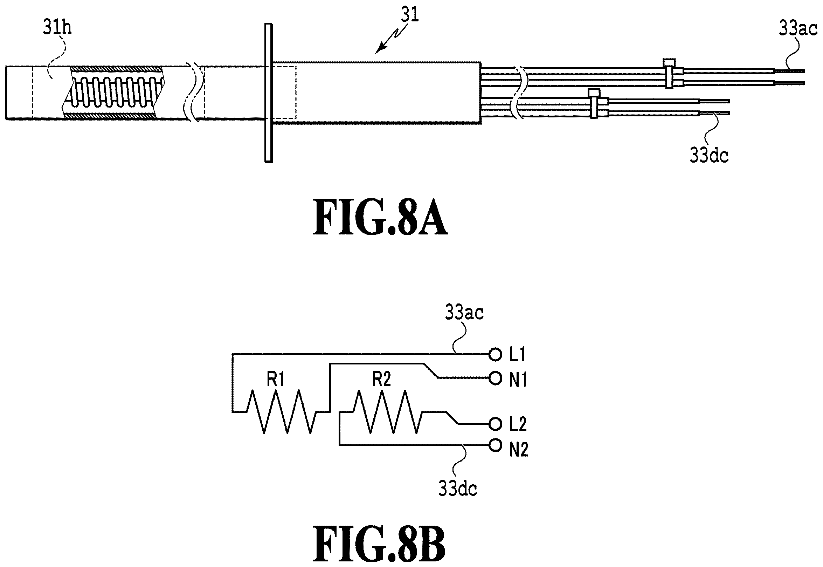

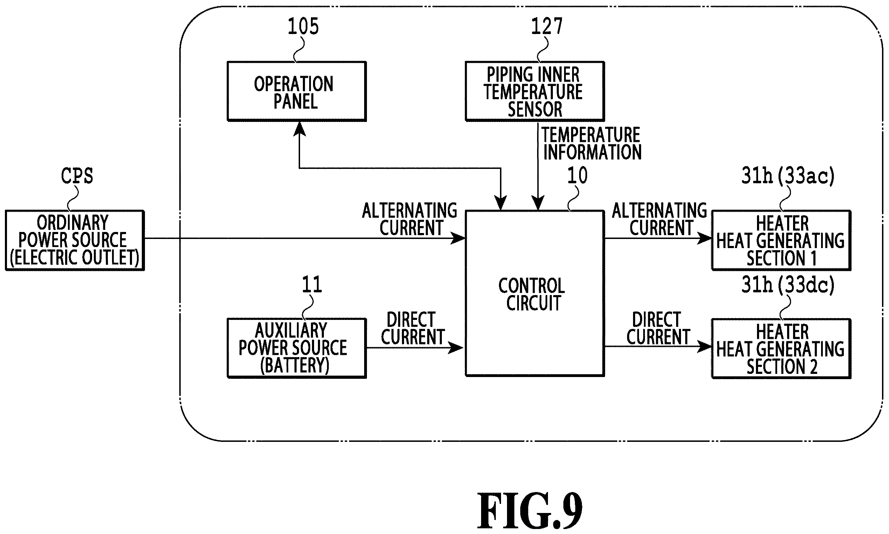

Next, Figure 8 and Figure 9 are views illustrating a blood purification apparatus according to a second embodiment of the present invention. Here, because the present embodiment is configured approximately the same as the embodiment described above, the same drawings are used, and similar constituent elements are denoted by the same reference numerals as in the above embodiment and characteristic portions are described (the same also applies with respect to another embodiment described hereinafter).

In Figure 8, instead of the heater 125 in the embodiment described above, the blood purification apparatus 100 includes a heater 31 having a heat generating section 31h installed in the introduction line 121 of the dialysis circuit 120. The heater 31 is constructed by containing, in the heat generating section 31h, a resistance component R1 around which is wound a nichrome wire or the like that generates heat when energized with AC power of an energizing circuit 33ac, and a resistance component R2 around which is wound a nichrome wire or the like that generates heat when energized with DC power of an energizing circuit 33dc.

As illustrated in Figure 9, the energizing circuits 33ac and 33dc are each individually connected to the control circuit 10 without an AC/DC converter or an inverter as well as a voltage conversion circuit or the like being interposed therebetween, and the heater 31 can be supplied with commercial AC power or with stored DC power of the battery 11. Here, although a case where the heater 31 includes the resistance components R1 and R2 as two types of heat generating elements is described as an example, the present invention is not limited thereto, and it need scarcely be said that, for example, a configuration may be adopted in which the heater 31 includes three or more separate heat generating elements.

### <Advantageous Effect of Second Embodiment>

As described above, in the blood purification apparatus 100 of the present embodiment, in addition to the operational advantages of the foregoing embodiment, a disinfection process with respect to the dialysis circuit 120 can be executed with a simpler configuration and simpler control.

### <Third Embodiment>

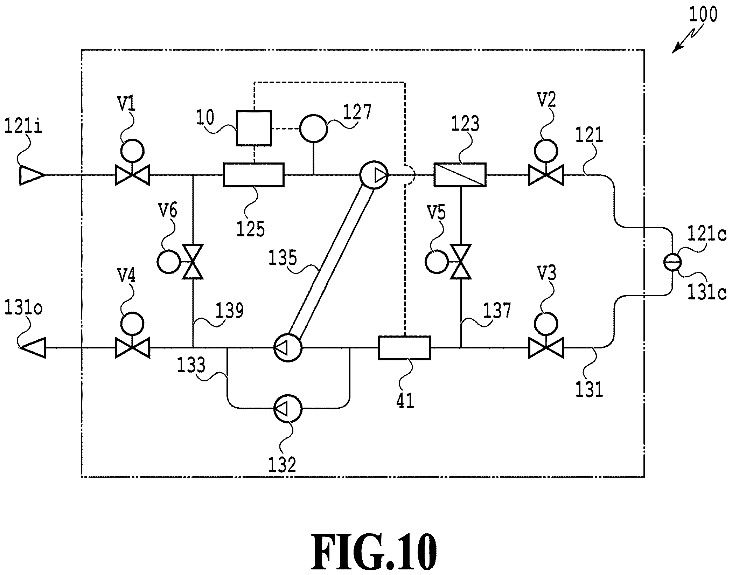

Next, Figure 10 is a view illustrating a blood purification apparatus according to a third embodiment of the present invention.

In Figure 10, in addition to the heater 125 arranged along the introduction line 121 of the dialysis circuit 120 in the embodiment described above, the blood purification apparatus 100 includes a heater 41 which is arranged along the drainage line 131 of the dialysis circuit 120 and which is connected to the control circuit 10. The heater 41 is arranged at a position that divides the route of the dialysis circuit 120 in two together with the heater 125, and is constructed so as to raise the temperature of the disinfection liquid circulating therein.

The heater 41 is arranged in the drainage line 131 which is separated from the heater 125 of the introduction line 121. Similarly to the heater 125, the control circuit 10 can supply commercial power and also power stored in the battery 11 to the heater 41 to heat and raise the temperature of the disinfection liquid flowing through the dialysis circuit 120. Here, a configuration may be adopted in which the heater 41 is utilized as a dedicated heating unit that operates using power stored in the battery 11, so that it may be constructed at a low cost, and in addition a configuration may be adopted in which the heater 125 is utilized as a dedicated heating unit that uses commercial power, so that it may be constructed at a lower cost. However, when taking into consideration the heating efficiency, it is advantageous to adopt the configuration described in the present embodiment. Note that, although a case in which one heater 41 is additionally installed in the dialysis circuit 12 is described as an example, the present invention is not limited thereto, and it need scarcely be said that, for example, heaters may be additionally installed so as to provide a heater at three or more locations including the heater 125.

By this means, the heaters 125 and 41 that are two heating units are provided by the dialysis circuit 120, and thus the temperature of the disinfection liquid in the drainage line 131 which is difficult to heat because it is at a remote position can be effectively raised and the disinfection liquid can be circulated toward the introduction line 121, and the temperature of the disinfection liquid can be caused to reach the target temperature Tt quickly in an efficient manner.

### <Advantageous Effect of Third Embodiment>

As described above, in the blood purification apparatus 100 of the present embodiment, in addition to the operational advantages of the embodiment described above, disinfection liquid in the dialysis circuit 120 can be heated more effectively and efficiently to carry out a disinfection process.

Here, although a case in which the heater 41 is used together with the heater 125 as shared heaters is described as an example in the present embodiment, the present invention is not limited thereto. For example, a configuration may be adopted in which commercial power is supplied to the heater 125 as a first heater unit, and power stored in the battery 11 is supplied to the heater 41 as a second heater unit.

### <Summary of Embodiments>

Next, matters specifying the invention that constitutes the invention of the present application are then described citing reference numerals and the like used in the foregoing embodiments. Note that, it need scarcely be said that the respective reference numerals and the like described below are used merely to describe constituent elements in the claims by citing the description of the embodiments, and are not intended to limit such constituent elements to specific members or the like.
[1] A blood purification apparatus (100) including a blood purifier (blood circuit 110, dialysis circuit 120, and dialyzer 130), the blood purification apparatus (100) including:
   a disinfector (dual pump 135 and control circuit 10) that causes a disinfection liquid to flow along a flow route of the blood purifier to disinfect the flow route, a heating unit (heater 125) that heats the disinfection liquid, and a controller (control circuit 10) that controls a blood purification treatment of the blood purifier and also controls a disinfection process of the disinfector and a heating process of the heating unit;
   wherein the heating unit is supplied with electric power from an auxiliary power source (battery 11) and generates heat in addition to being supplied with electric power from a commercial power source and generating heat to heat the disinfection liquid.
[2] The blood purification apparatus according to [1], wherein the auxiliary power source is shared by the blood purifier and the heating unit.
[3] The blood purification apparatus according to [1] or [2], wherein the heating unit includes a first heater unit (heater 125) that is supplied with electric power from a commercial power source and generates heat, and a second heater unit (heater 41) that is supplied with electric power from the auxiliary power source and generate heats.
[4] The blood purification apparatus according to [1] or [2], wherein the heating unit includes a shared heater unit (heater 125, heater 41) that is supplied with electric power from a commercial power source and electric power from the auxiliary power source and generates heat.
[5] The blood purification apparatus according to any one of [1] to [4], wherein the heating unit is arranged at a plurality of places within the flow route.
[6] The blood purification apparatus according to any one of [1] to [5], including a temperature sensor (thermistor 127) for detecting a temperature of a disinfection liquid within the flow route on a downstream side of the heating unit within the flow route,
   wherein the controller controls a heating process of the heating unit so as to maintain a temperature of a disinfection liquid within the flow route at a desired temperature or higher for a desired time period or longer which are set in advance, based on detection information of the temperature sensor.
[7] The blood purification apparatus according to [6], wherein the controller supplies electric power to the heating unit according to a temperature of disinfection liquid within the flow route based on the detection information of the temperature sensor.
[8] The blood purification apparatus according to any one of [1] to [7], including a battery 11 as the auxiliary power source and including a detector (control circuit 10) for detecting a remaining amount of stored electricity in the battery,
   wherein the controller performs control processing for at least one of stopping heating and status notification according to at least one of a remaining amount of stored electricity in the battery and a deterioration status of the battery.

In the case of the above [1], during a disinfection process within the flow route of the blood purifier, the disinfection liquid can be heated by supplying electric power to the heating unit from the auxiliary power source in addition to the commercial power source, and hence the disinfection liquid can be easily heated and the disinfection process can be quickly completed.

In the case of the above [2], the auxiliary power source of the blood purifier can be shared with the heating unit, and a quick disinfection process can be realized at low cost.

In the case of the above [3] to [5], the inside of the flow route of the blood purifier can be appropriately heated and a disinfection process can be effectively executed.

In the case of the above [6] and [7], the temperature within the flow route of the blood purifier can be accurately measured and a disinfection process can be efficiently executed.

In the case of the above [8], according to the remaining amount of stored electricity in the battery or the deterioration status of the battery, heating can be stopped or the status can be notified so that an appropriate countermeasure can be taken.

The scope of the present invention is not limited to the exemplary embodiments illustrated in the drawings and described above, and includes all embodiments that bring equivalent effects at which the present invention aims. Moreover, the scope of the present invention is not limited to combinations of features of the invention defined by each claim, but may include all desired combinations of specific features among the respective features described heretofore.

### Reference Signs List

- 10: control circuit
- 11: battery
- 21, 31, 41, 125: heater
- 100: blood purification apparatus
- 105: operation panel
- 110: blood circuit
- 111: blood removal line
- 113: blood return line
- 115: blood pump
- 120: dialysis circuit
- 121: introduction line
- 121c, 131c: connecting tool
- 123: filtration filter
- 127: thermistor
- 130: dialyzer
- 131: drainage line
- 132: water removal pump
- 133: detour line
- 135: dual pump
- 137, 139: bypass line
- CPS: commercial power source

## Claims

1. A blood purification apparatus including a blood purifier, the blood purification apparatus comprising:
a disinfector that causes a disinfection liquid to flow along a flow route of the blood purifier to disinfect the flow route, a heating unit that heats the disinfection liquid, and a controller that controls a blood purification treatment of the blood purifier and also controls a disinfection process of the disinfector and a heating process of the heating unit,
wherein the heating unit is supplied with electric power from an auxiliary power source and generates heat in addition to being supplied with electric power from a commercial power source and generating heat to heat the disinfection liquid.

2. The blood purification apparatus according to claim 1, wherein:
the auxiliary power source is shared by the blood purifier and the heating unit.

3. The blood purification apparatus according to claim 1 or claim 2, wherein:
the heating unit comprises a first heater unit that is supplied with electric power from a commercial power source and generates heat, and a second heater unit that is supplied with electric power from the auxiliary power source and generates heat.

4. The blood purification apparatus according to claim 1 or claim 2, wherein:
the heating unit comprises a shared heater unit that is supplied with electric power from a commercial power source and electric power from the auxiliary power source and generates heat.

5. The blood purification apparatus according to any one of claim 1 to claim 4, wherein:
the heating unit is arranged at a plurality of places within the flow route.

6. The blood purification apparatus according to any one of claim 1 to claim 5, comprising:
a temperature sensor for detecting a temperature of a disinfection liquid within the flow route on a downstream side of the heating unit within the flow route,
wherein the controller controls a heating process of the heating unit so as to maintain a temperature of a disinfection liquid within the flow route at a desired temperature or higher for a desired time period or longer which are set in advance, based on detection information of the temperature sensor.

7. The blood purification apparatus according to claim 6, wherein:
the controller supplies electric power to the heating unit according to a temperature of a disinfection liquid within the flow route based on the detection information of the temperature sensor.

8. The blood purification apparatus according to any one of claim 1 to claim 7, comprising a battery as the auxiliary power source and comprising a detector for detecting a remaining amount of stored electricity in the battery,
wherein the controller performs control processing for at least one of stopping heating and status notification according to at least one of a remaining amount of stored electricity in the battery and a deterioration status of the battery.
